# EUROPÄISCHE PATENTSCHRIFT

(11) **EP 2 119 406 B1**
(45) Veröffentlichungstag und Bekanntmachung des Hinweises auf die Patenterteilung: **28.12.2011**
(21) Anmeldenummer: 08156089.8
(22) Anmeldetag: 13.05.2008
(51) Int. Cl.: A61B 17/70

(54) **Pedikelschraube mit einer Verschlusseinrichtung**
Pedicle screw with a locking device
Vis pédiculaire dotée d'un dispositif de fermeture

(43) Veröffentlichungstag der Anmeldung: 18.11.2009
(73) Patentinhaber: Spinelab AG, 8406 Winterthur (CH)
(72) Erfinder: Zehnder, Thomas, 8806, Bäch (CH)
(74) Vertreter: BOVARD AG

(56) Entgegenhaltungen:
- EP-A- 1 795 134
- WO-A-2007/089984
- WO-A-2007/097905
- WO-A-2007/122494
- DE-A1- 10 005 134
- US-A- 5 536 268
- US-A1- 2004 260 283

## Beschreibung

Die vorliegende Erfindung bezieht sich auf eine Pedikelschraube mit einer Verschlusseinrichtung, in welcher ein aus einem elastischen Kunststoff bestehender Stab aufgenommen und fixiert ist, zur Stabilisierung einer Wirbelsäule, umfassend einen Einschraubteil, einen am Einschraubteil angebrachten Kopfteil, eine im Kopfteil angeordnete, durch zwei Schenkel gebildete U-förmige Ausnehmung, in welche der zu haltende Stab einsetzbar ist, ein Verriegelungselement, mit welchem die U-förmige Ausnehmung verschliessbar ist, eine Spannschraube und ein Einsatzelement, das auf den Stab aufsetzbar ist und diesen mindestens teilweise umschliesst, und der Stab im eingespannten Zustand zwischen einer ersten Spannfläche und einer zweiten Spannfläche gehalten ist, und das Einsatzelement mit Anschlagflächen ausgestattet ist, durch welche beim Spannvorgang die durch die Spannschraube auf das Einsatzelement übertragene Spannbewegung gegen den Stab hin begrenzt ist.

Derartige Pedikelschrauben mit einer Verschlusseinrichtung zur Aufnahme und Fixierung eines aus einem elastischen Kunststoff bestehenden Stabes sind in vielfältiger Weise bekannt. Die Pedikelschrauben werden jeweils in einen Wirbelkörper einer zu stabilisierenden Wirbelsäule eines Patienten eingeschraubt, in die Pedikelschrauben wird der Stab eingesetzt, der Aufnahmebereich der Pedikelschraube für den Stab wird mit einer Verschlusseinrichtung abgeschlossen, mittels der in die Verschlusseinrichtung eingesetzten Spannschraube wird der Stab in der jeweiligen Pedikelschraube festgeklemmt, wozu in vorteilhafter Weise zwischen Spannschraube und Stab ein Einsatzelement eingesetzt wird. Durch die Verwendung eines aus einem elastischen Kunststoff bestehenden Stabes wird erreicht, dass die einzelnen Wirbel der so stabilisierten Wirbelsäule nicht vollständig fixiert werden, wie dies bei der Verwendung eines Stabes aus beispielsweise Stahl auftreten würde, eine geringe Bewegung zwischen den einzelnen stabilisierten Wirbeln ist möglich, wodurch vermieden wird, dass die Wirbel durch eine Verknöcherung sich miteinander verbinden.

Die Aufnahme und Fixierung des aus einem elastischen Kunststoff bestehenden Stabes in der Pedikelschraube ist entscheidend für die langzeitliche gleichbleibende stabilisierende Unterstützung einer entsprechend ausgerüsteten Wirbelsäule. Bei den bekannten Systemen, welche einen Stab aus einem elastischen Kunststoff verwenden, wird der Stab mit einer grossen Spannkraft in der Pedikelschraube fixiert und gehalten. Hierbei wird die auf den elastischen Stab wirkende Spannkraft durch einschrauben der Spannschraube in mehr oder weniger unkontrollierter Weise aufgebracht. Es ist bekannt, dass elastische Kunststoffe unter Dauerdruck zum Fliessen neigen, in den vorliegenden Fällen kann somit die ursprünglich aufgebrachte Spannkraft durch Fliessen des Kunststoffes abgebaut werden, die Stabilität der die Wirbelsäule stützenden Einrichtung ist gefährdet.

Aus der EP-A-1 795 134 ist eine Einrichtung bekannt, bei welcher die Spannkraft begrenzt ist, wodurch eine Verbesserung der Fliessneigung erreicht wird.

Die Aufgabe der vorliegenden Erfindung besteht somit darin, eine Pedikelschraube mit einer Verschlusseinrichtung zur Aufnahme und Fixierung eines aus einem elastischen Kunststoff bestehenden Stabes zur Stabilisierung einer Wirbelsäule so auszugestalten, dass eine vordefinierte Spannkraft auf den Stab aufgebracht werden kann und dadurch das Risiko des Fliessens des Kunststoffes unter Dauerdruck eingeschränkt werden kann und an den Randbereichen Spitzenbelastungen zumindest reduziert werden können.

Erfindungsgemäss erfolgt die Lösung dieser Aufgabe dadurch, dass die erste Spannfläche und/oder die zweite Spannfläche mindestens teilweise mit Erhöhungen und/oder mit Vertiefungen ausgestattet sind und die Breite der an den Spannflächen angebrachten Erhöhungen in Stabrichtung gegen die Randbereiche hin abnehmend und/oder die Breite der an den Spannflächen angebrachten Vertiefungen in Stabrichtung gegen die Randbereiche hin zunehmend sind.

Mit dieser Ausgestaltung wird erreicht, dass die auf den Stab wirkende Spannkraft vorgegeben ist, da das Einsatzelement den Stab nur soweit elastisch verformt, bis das Einsatzelement mit den Anschlagflächen zur Auflage kommt, ein zu starkes Spannen der Spannschraube wird dadurch vermieden, die auf den Stab wirkende Spannkraft bleibt gleich.

Durch die bereichsweise stärkere Verformung des Stabes im Einspannbereich kann eine bessere Fixierung erreicht werden.

Zudem wird erreicht, dass bei Zugbeanspruchung des Stabes die Kräfte optimal auf die Spannflächen des Einsatzelemerttes übertragen werden und an den Randbereichen keine Spitzenbelastungen auftreten.

Das Einsatzelement kann als Schiebeteil ausgebildet sein, welches geführt zwischen die beiden Schenkel der U-förmigen Ausnehmung einsetzbar ist, welche Schenkel mit jeweils einer innenseitig vorstehenden Schulter ausgestattet sind, auf welchen sich die Anschlagflächen des Schiebeteils abstützen, wobei die erste Spannfläche durch die gegen den Stab gerichtete Fläche des Schiebeteils und die zweite Spannfläche durch den Grund der U-förmigen Ausnehmung im Kopfteil gebildet sind, was eine optimale Fixierung des Stabes ergibt.

Das Einsatzelement kann auch aus einer im wesentlichen hohlzylinderförmigen Hülse gebildet sein, in welche der Stab einsetzbar ist, welche Hülse aus einer ersten Halbschale und einer zweiten Halbschale zusammengesetzt ist und welche beiden Halbschalen über die Spannschraube gegeneinander spannbar sind, wobei an der ersten Halbschale die Anschlagflächen angebracht sind, welche auf entsprechend an der zweiten Halbschale angebrachte Stützflächen abstützbar sind. Dadurch kann die Pedikelschraube sehr einfach gestattet sein. Die Innenseite der ersten Halbschale bildet hierbei die erste Spannfläche, die Innenseite der zweiten Halbschale bildet die zweite Spannfläche.

In vorteilhafter Weise sind die erste Halbschale und die zweite Halbschale entlang der einen Trennlinie gelenkig miteinander verbunden, und die erste Halbschale weist auf der aufschwenkbaren Seite mindestens eine Lasche auf, welche in entsprechend an der aufschwenkbaren Seite der zweiten Halbschale angebrachte Ausnehmungen eingreift. Dadurch erhält man eine einfache Handhabung der Hülse.

In vorteilhafter Weise weist der in die Pedikelschraube einsetzbare Stab eine zu den Spannflächen mindestens teilweise komplementäre Oberfläche auf, wodurch mindestens teilweise eine formschlüssige Verbindung erhalten wird.

In vorteilhafter Weise besteht der Stab aus einem biokompatiblen Kunststoff auf Basis von Polyurethan und die Pedikelschraube mit Verriegelungselement und Einsatzelement aus einer Titanlegierung.

In vorteilhafter Weise kann der in die Pedikelschraube einzusetzende Stab erwärmt werden, wodurch einerseits eine Verminderung der Biegesteifigkeit erreicht wird, andererseits kann der eine glatte Oberfläche aufweisende Stab durch die strukturierten Oberflächen der Spannflächen im eingespannten Zustand eine plastische Verformung erfahren, wodurch man eine formschlüssige Verbindung erhält.

Ausführungsformen der Erfindung werden nachfolgend anhand der beliegenden Zeichnung beispielhaft näher erläutert.

Die Ausführungsformen von den Figuren 7-11, 14-16 fallen nicht unter die Ansprüche.

Es zeigt:
Fig. 1 eine Schnittdarstellung durch eine Pedikelschraube mit eingesetztem Stab und aufgesetztem Verriegelungselement, im nicht gespannten Zustand;
Fig. 2 eine Schnittdarstellung durch die Pedikelschraube gemäss Fig. 1, im gespannten Zustand;
Fig. 3 in räumlicher Darstellung eine Pedikelschraube mit eingesetztem Stab, in welchem das Einsatzelement als hohlzylinderförmige Hülse ausgebildet ist;
Fig. 4 eine Schnittdarstellung durch die Pedikelschraube gemäss Fig. 3;
Fig. 5 in räumlicher Darstellung eine hohlzylinderförmige Hülse im geschlossenen Zustand;
Fig. 6 in räumlicher Darstellung die hohlzylinderförmige Hülse beim Aufsetzen auf einen Stab;
Fig. 7 eine Schnittdarstellung durch eine hohlzylinderförmige Hülse;
Fig. 8 in räumlicher Darstellung eine hohlzylinderförmige Hülse mit auf den Spannflächen angebrachten Erhöhungen;
Fig. 9 im Schnitt eine mit Rippen und Rillen versehene Spannfläche eines Einsatzelementes und einem eine komplementäre Oberfläche aufweisenden Stab;
Fig. 10 im Schnitt eine mit Rippen und Rillen versehende Spannfläche eines Einsatzelementes mit einem Stab, der eine teilweise komplementäre Oberfläche aufweist;
Fig. 11 im Schnitt eine mit Rippen und Rillen versehene Spannfläche eines Einsatzelementes und einem eine glatte Oberfläche aufweisenden Stab;
Fig. 12 im Schnitt eine mit Rippen und Rillen versehende Spannfläche eines Einsatzelementes und einem eine komplementäre Oberfläche aufweisenden Stab, wobei die Breite der Rippen der Spannfläche gegen den Randbereich des Einsatzelementes hin abnehmend ist;
Fig. 13 im Schnitt das Einsatzelemente gemäss Fig. 12, wobei der hier eingesetzte Stab eine teilweise komplementäre Oberfläche zu der Spannfläche aufweist;
Fig. 14 im Schnitt einen eine glatte Oberfläche aufweisenden Stab und das Einsatzelement mit einer Rippen und Rille aufweisenden Spannfläche vor dem Spannvorgang;
Fig. 15 das Einsatzelement gemäss Fig. 14 im gespannten Zustand, wobei der Stab vor dem Spannvorgang erwärmt wurde; und
Fig. 16 im Schnitt das Einsatzelement gemäss Fig. 14 und Fig. 15, nachdem es wieder gelöst worden ist.

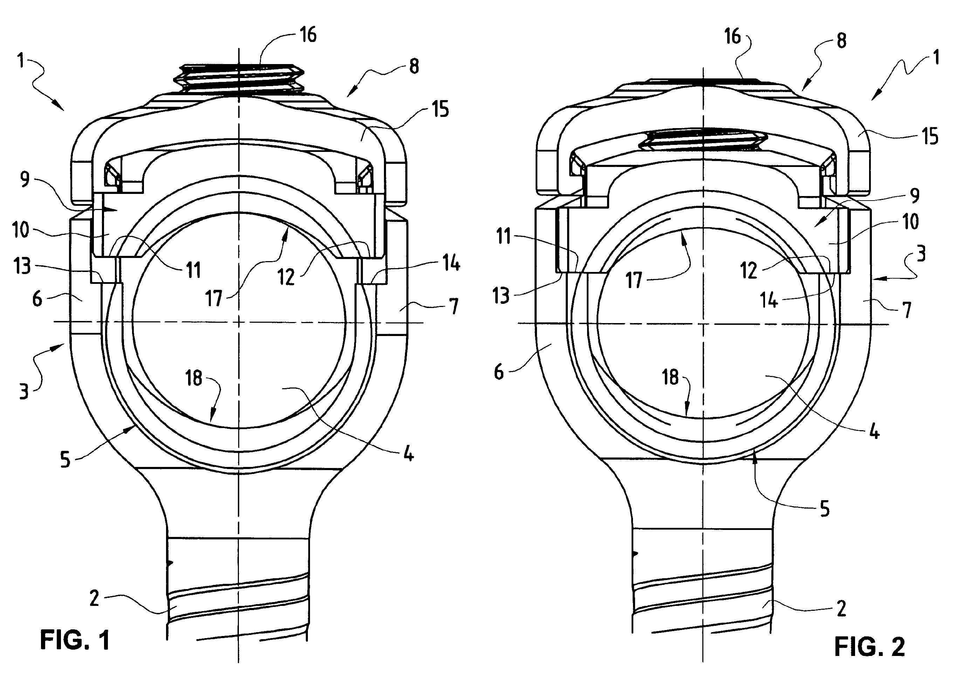

Aus den Fig. 1 und 2 ist jeweils eine Pedikelschraube 1 ersichtlich, die in bekannter Weise aus einem nur teilweise dargestellten Einschraubteil 2 und einem am Einschraubteil 2 angebrachten Kopfteil 3 besteht. Diese Pedikelschraube 1 kann in bekannter Weise in jeweils einen Wirbelkörper einer zu stabilisierenden Wirbelsäule eingeschraubt werden. Der Kopfteil 3 ist zur Aufnahme und Fixierung eines Stabes 4 ausgestaltet. Hierzu weist der Kopfteil 3 eine U-förmige Ausnehmung 5 auf, die durch zwei Schenkel 6 und 7 gebildet ist. In diese U-förmige Ausnehmung 5 kann der Stab 4 eingelegt werden.

Auf die beiden Schenkel 6 und 7 wird eine Verschlusseinrichtung 8 aufgesetzt, die mit den beiden Schenkeln in bekannter Weise verriegelt wird. Im hier dargestellten Ausführungsbeispiel wird zwischen die Verschlusseinrichtung 8 und den Stab 4 ein Einsatzelement 9 eingesetzt, das als Schiebeteil 10 ausgebildet ist, welches geführt zwischen die beiden Schenkel 6 und 7 der U-förmigen Ausnehmung 5 auf den Stab aufgesetzt wird. Dieses Schiebeteil 10 weist zwei Anschlagflächen 11 und 12 auf, an den beiden Schenkeln 6 und 7 ist jeweils eine Schulter 13 und 14 angebracht.

Zur Fixierung des Stabes 4 in der Pedikelschraube 1 wird die im Verriegelungselement 15 der Verschlusseinrichtung 8 eingesetzte Spannschraube 16 angezogen. Wie aus Fig. 2 ersichtlich ist, wird hierbei der Schiebeteil 10 bezüglich des Verriegelungselementes 15 gegen den Stab 4 hin abgesenkt, die Spannschraube 16 wird so lang verdreht, bis die Anschlagflächen 11 und 12 des Schiebeteils 10 auf der jeweiligen Schulter 13 und 14 der beiden Schenkel 6 und 7 zur Auflage kommt. Der Stab 4 wird somit zwischen einer ersten Spannfläche 17, die am Schiebeteil 10 angeformt ist, und einer zweiten Spannfläche 18, die im Grund der U-förmigen Ausnehmung 5 des Kopfteils 3 gebildet ist, eingespannt.

Durch diese Ausgestaltung der Elemente, mit welchen der Stab 4 eingespannt wird, wird erreicht, dass die Einspannung des Stabes immer gleich ist, unabhängig davon, wie stark die Spannschraube verdreht wird. Wesentlich ist, dass die beiden Anschlagflächen 11 und 12 sich auf den Schultern 13 und 14 abstützen. Ein zu starkes Spannen des aus einem elastischen Kunststoff, beispielsweise einem biokompatiblen Kunststoff auf Basis von Polyurethan, bestehenden Stabes wird dadurch verunmöglicht.

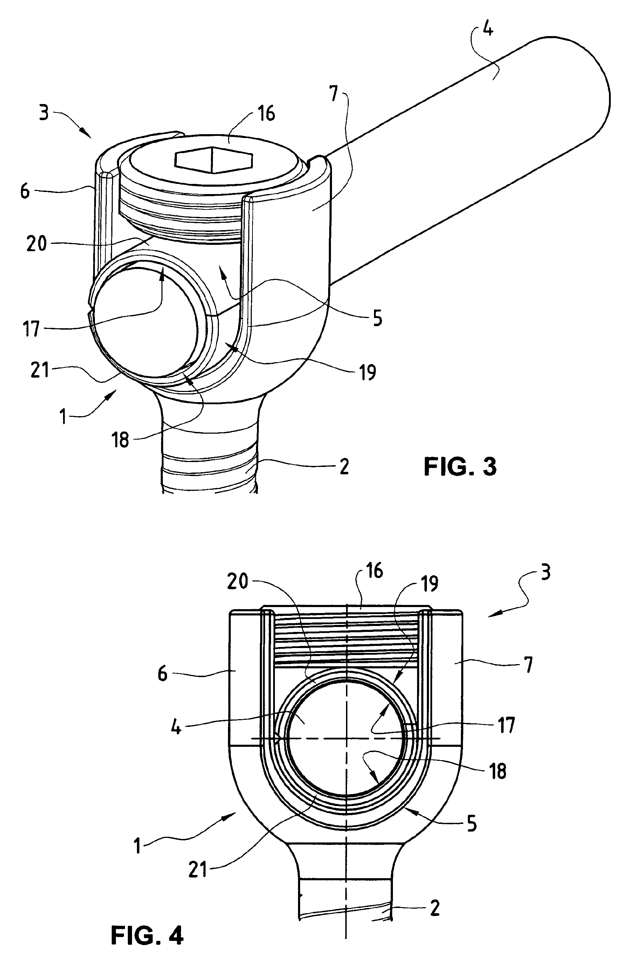

Aus den Fig. 3 und 4 ist eine weitere Ausführungsform der vorliegenden Erfindung ersichtlich. In der folgenden Beschreibung werden für gleiche Teile die gleichen Bezugszeichen verwendet, wie im vorhergehenden Ausführungsbeispiel. Die hier dargestellte Pedikelschraube 1 umfasst wiederum einen Einschraubteil 2 und einen Kopfteil 3, wobei der Einschraubteil 2 wiederum in einen jeweiligen Wirbelkörper einer zu stabilisierenden Wirbelsäule eingeschraubt wird. Der Kopfteil 3 ist wiederum mit einer U-förmigen Ausnehmung 5 ausgestattet, gebildet durch zwei Schenkel 6 und 7. In die U-förmige Ausnehmung 5 eingelegt werden kann der zu haltende und zu fixierende Stab 4.

Um den Stab 4 herum gelegt ist im Bereich der U-förmigen Ausnehmung 5 eine hohlzylinderförmige Hülse 19, die als Einsatzelement 9 dient. Diese hohlzylinderförmige Hülse 19 ist aus einer ersten Halbschale 20 und einer zweiten Halbschale 21 zusammengesetzt, wie nachfolgend noch im Detail beschrieben wird.

Der Stab 4 mit der darauf aufgesetzten Hülse 19 wird in die U-förmige Ausnehmung 5 des Kopfteils 3 der Pedikelschraube 1 eingesetzt, zwischen den beiden Schenkeln 6 und 7 ist in bekannter Weise ein Gewinde angeordnet, in welches die Spannschraube 16 eingeschraubt wird. Der Stab 4 wird zwischen der ersten Halbschale 20 und der zweiten Halbschale 21 der hohlzylinderförmigen Hülse 19 fixiert. Die Innenseite der ersten Halbschale 20 bildet die erste Spannfläche 17, während die Innenseite der zweiten Halbschale 21 die zweite Spannfläche 18 bildet. Diese hohlzylinderförmige Hülse 19 bildet somit das Einsatzelement 9, mittels welchem der Stab 4 in der Pedikelschraube 1 gehalten wird.

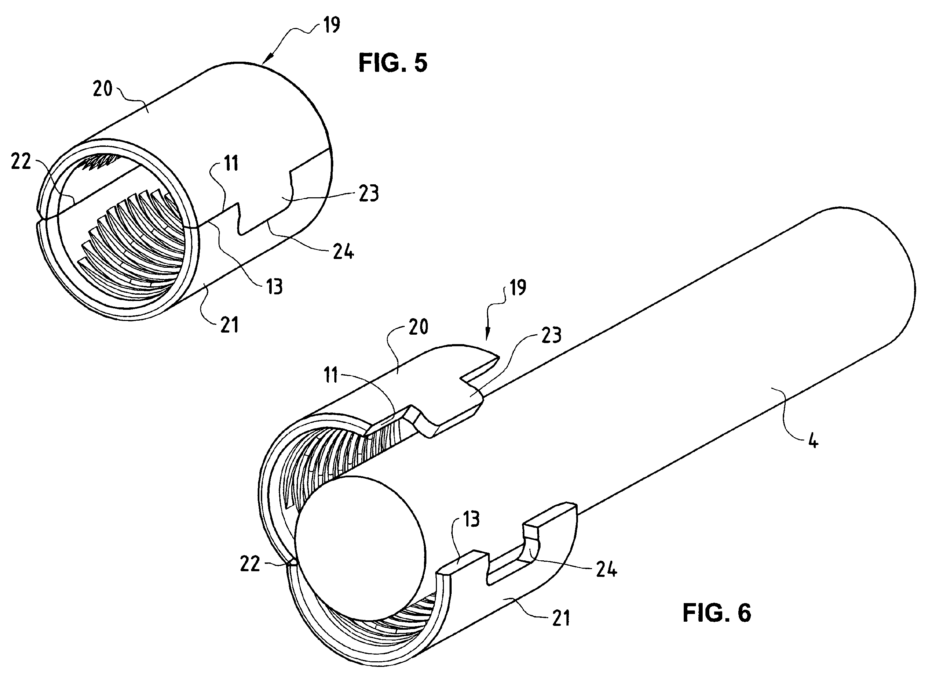

Aus den Fig. 5 und 6 ist die hohlzylinderförmige Hülse 19 ersichtlich. Hierbei sind die erste Halbschale 20 und die zweite Halbschale 21 entlang der einen Trennlinie 22 in bekannter Weise gelenkig miteinander verbunden. Dadurch kann die erste Halbschale 20 und die zweite Halbschale 21 um dieses Gelenk aufgeschwenkt werden, wie dies aus Fig. 6 ersichtlich ist. Durch dieses Aufschwenken kann die hohlzylinderförmige Hülse 19 in einfacher Weise auf den Stab 4 aufgesetzt werden.

Auf der aufschwenkbaren Seite ist an der ersten Halbschale 20 eine Lasche 23 angebracht, während die zweite Halbschale 21 mit einer entsprechenden Ausnehmung 24 versehen ist. Im zugeschwenkten Zustand der hohlzylinderförmigen Hülse 19 kommt die Lasche 23 in die Ausnehmung 24 zu liegen. Die Lasche 23 und die Ausnehmung 24 können so ausgebildet sein, dass eine Art Schnappverschluss erreicht wird, so dass die hohlzylinderförmige Hülse 19 auf den Stab aufgesetzt und zugeschwenkt werden kann, bis der Schnappverschluss eingreift, hierbei kann dieser Schnappverschluss so ausgebildet sein, dass mit eingeklinktem Schnappverschluss die hohlzylinderförmige Hülse 19 auf dem Stab 4 noch in Längsrichtung verschiebbar ist.

Die erste Halbschale 20 weist auf der aufschwenkbaren Seite die erste Anschlagfläche 11 auf, die entsprechende Seite der zweiten Halbschale 21 bildet die Schulter 13, auf welcher die Anschlagfläche 11 im verspannten Zustand zur Anlage kommt. In diesem gespannten Zustand, wie er in den Fig. 3 und 4 dargestellt ist, stützt sich somit die Anschlagfläche 11 der ersten Halbschale 20 auf der Schulter 13 der zweiten Halbschale 21 ab. Dadurch wird der voll verspannte Zustand des Stabes 4 erreicht, ein weiteres Zupressen der Hülse, die aus einer metallischen Legierung, insbesondere einer Titanlegierung hergestellt ist, erfolgt nicht, der Stab wird somit in einem vorbestimmten Spannungszustand gehalten.

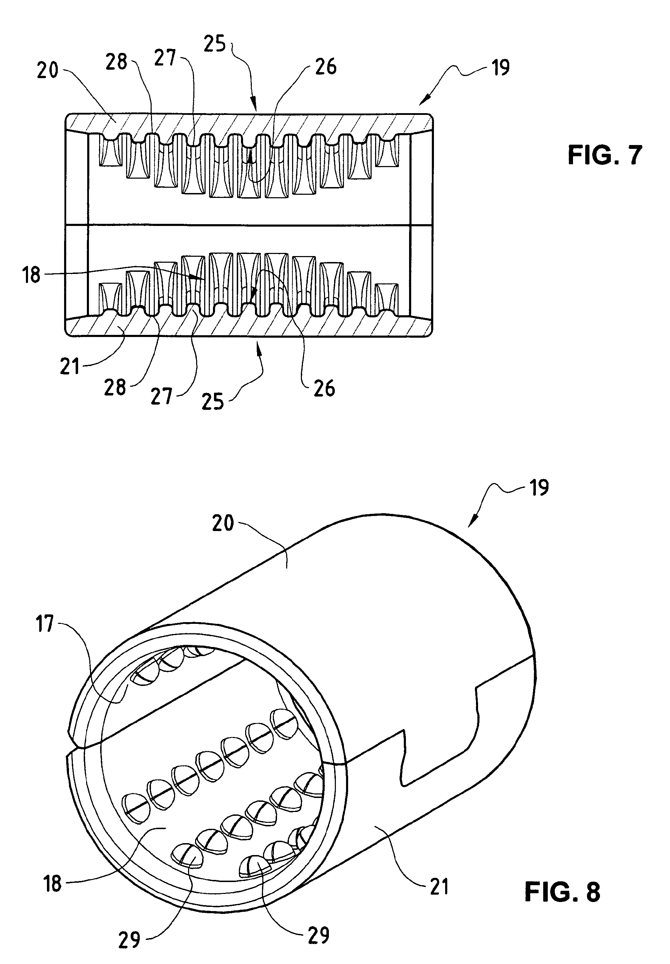

Wie aus Fig. 7 ersichtlich ist, kann die erste Spannfläche 17 und die zweite Spannfläche 18 der hohlzylinderförmigen Hülse 19 in deren mittleren Bereich 25 eine gegen den eingesetzten Stab 4 vorstehende Wölbung 26 aufweisen. Dadurch wird erreicht, dass der eingespannte Stab in diesem mittleren Bereich 25 stärker zusammengepresst wird, als in den randseitigen Bereichen, wodurch eine übermässige Spitzenspannung am Randbereich der beiden Spannflächen 17 und 18 vermieden wird. Diese Wölbungen 26 können eine glatte Oberfläche aufweisen, sie können aber auch, wie dies in Fig. 7 dargestellt ist, mit Erhöhungen 27 und Vertiefungen 28 ausgebildet sein, diese Erhöhungen 27 und Vertiefungen 28 können als Rippen und Rillen ausgebildet sein, welche im wesentlichen quer zur Stabrichtung verlaufen.

Wie aus Fig. 8 ersichtlich ist, können die erste Spannfläche 17 und die zweite Spannfläche 18 der hohlzylinderförmigen Hülse 19 auch mit vorstehenden Nocken 29 ausgestattet sein.

Die in Fig. 7 und Fig. 8 dargestellten Ausgestaltungen der ersten Spannfläche 17 und der zweiten Spannfläche 18 der hohlzylinderförmigen Hülse 19 sind selbstverständlich auch in der ersten Spannfläche 17 und der zweiten Spannfläche 18 der Ausführungsformen, wie sie in Fig. 1 und 2 dargestellt sind, anwendbar.

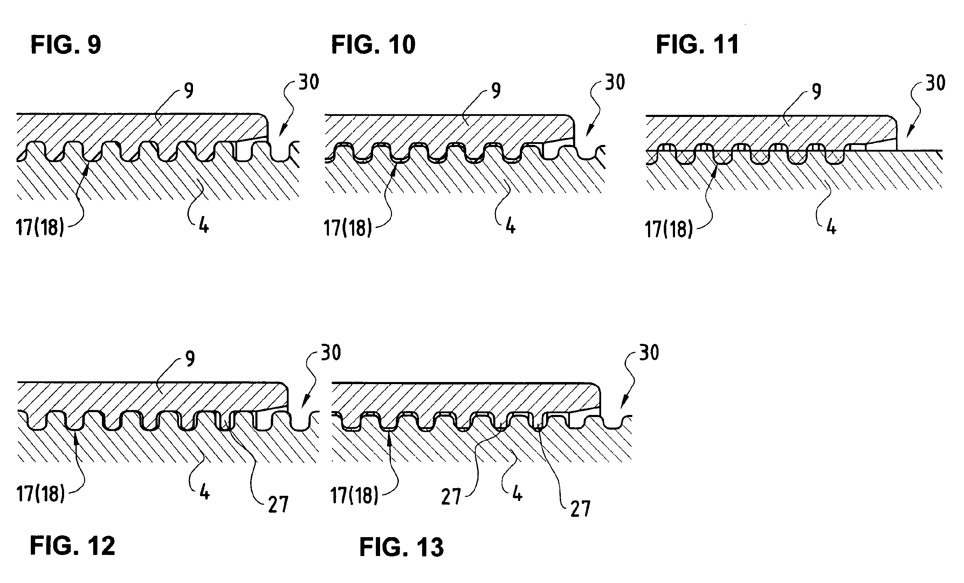

Der Stab 4 kann, wie dies in Fig. 9 dargestellt ist, eine Oberfläche 30 aufweisen, die komplementär zur ersten Spannfläche 17 und zur zweiten Spannfläche 18, mit welcher der Stab gehalten wird, ausgebildet ist. Dadurch erhält man zwischen den beiden Spannflächen 17 und 18 und dem Stab 4 eine volle formschlüssige Verbindung.

Wie aus Fig. 10 ersichtlich ist, kann die Oberfläche 30 des Stabes 4 eine zu den Spannflächen 17 und 18 teilweise komplementäre Struktur aufweise, man erhält dann eine teilweise formschlüssige Verbindung zwischen den Spannflächen 17 und 18 und dem Stab 4.

Es ist auch denkbar, wie dies in Fig. 11 ersichtlich ist, den Stab mit einer glatten Oberfläche 30 zu belassen, so dass im zugespannten Zustand die Erhöhungen der mit Erhöhungen und Vertiefungen ausgestatteten Spannflächen 17 und 18 teilweise in die Oberfläche des ursprünglich glatten Stabes 4 eindringen und dadurch das Festhalten des Stabes verbessert wird. Die Oberflächenstrukturen der Spannflächen 17 und 18 müssen hierbei so ausgestaltet sein, dass der Stab durch die Erhöhungen keine die Staboberfläche schneidende Verletzungen erfährt, sondern lediglich eingepresst wird.

Aus Fig. 12 ist eine Ausgestaltung ersichtlich, bei welcher die Oberfläche 30 des Stabes 4 eine komplementäre Form zu der Ausgestaltung der Spannflächen 17 und 18 aufweist. Die Erhöhungen 27 der Spannflächen 17 und 18 weisen in Stabrichtung gegen die Randbereiche der Spannflächen 17 und 18 hin eine abnehmende Breite auf. Dadurch wird erreicht, dass eine Zugbeanspruchung auf den Stab 4 nicht ausschliesslich am Randbereich auf den entsprechenden Spannkörper übertragen wird, sondern sich über eine relativ grosse Länge des Spannkörpers erstreckt.

Fig. 13 zeigt eine gleiche Ausgestaltung der Spannflächen 17 und 18, wobei der Stab 4 aber nur eine teilweise komplementäre Oberfläche 30 zu den Spannflächen 17 und 18 aufweist. Hier wird aber derselbe Effekt erreicht, wie er zur Ausgestaltung gemäss Fig. 12 beschrieben worden ist.

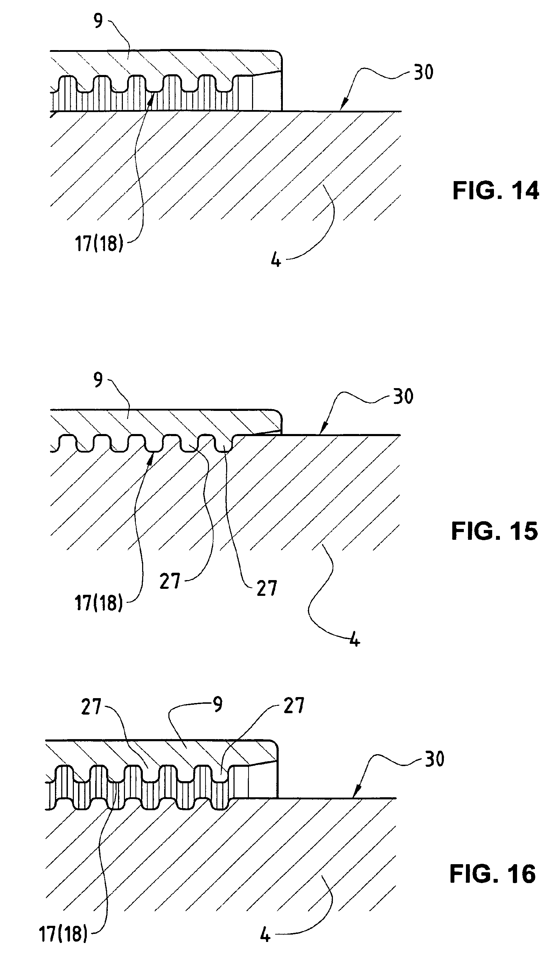

Fig. 14 zeigt einen Stab 4 mit glatter Oberfläche 30. Die Spannflächen 17 und 18 sind mit Rippen und Rillen ausgestattet. Hierbei ist der Stab 4 noch nicht zwischen die Spannflächen 17, 18 eingespannt. Der Stab kann vor dem Einsatz in die in die Wirbelsäule eingesetzten Pedikelschrauben vorgängig erwärmt werden, zum Beispiel durch Wasserbad, Dampf, Ofen oder ähnlichem. Der erwärmte Stab lässt sich dadurch leichter biegen und somit einfacher in die Pedikelschrauben einsetzen. Beim Spannen des Stabes zwischen den Spannflächen 17 und 18, wenn diese mit Rippen und Rillen versehen sind, dringen die Rippen in die weichere Oberfläche 30 des erwärmten Stabes 4 ein. Man erhält dadurch praktisch eine formschlüssige Verbindung zwischen den Spannflächen 17 und 18 und dem Stab 4, wie dies aus Fig. 15 ersichtlich ist.

Wenn sich der Stab 4 abgekühlt hat, und wenn die Spannflächen 17 und 18 von der Oberfläche 30 des Stabes abgehoben worden sind, verbleiben auf der Staboberfläche 30 Strukturen, die im Wesentlichen der Oberflächenstruktur der Spannflächen 17 und 18 entsprechen. Dies zeigt, dass man durch Erwärmen des Stabes quasi eine formschlüssige Verbindung zwischen Stab und Spannflächen erreichen kann. Beim Erwärmen des Stabes, der aus einem biokompatiblen Kunststoff auf Basis von Polyurethan hergestellt ist, soll die so genannte Glasübergangstemperatur (Vicat Softening Temparature) nicht überschritten werden, da sonst die Materialeigenschaften grundsätzlich geändert werden können. Diese Glasübergangstemperatur ist für verschiedene Materialien, wie bekannt ist, unterschiedlich.

Mit dieser erfindungsgemässen Ausgestaltung erhält man eine optimale Verbindung zwischen dem aus einem elastischen Kunststoff bestehenden Stab und den jeweiligen Pedikelschrauben.

## Patentansprüche

1. Pedikelschraube mit einer Verschlusseinrichtung (8), in welcher ein aus einem elastischen Kunststoff bestehender Stab (4) aufgenommen und fixiert ist, zur Stabilisierung einer Wirbelsäule, umfassend einen Einschraubteil (2), einen am Einschraubteil (2) angebrachten Kopfteil (3), eine im Kopfteil (3) angeordnete, durch zwei Schenkel (6, 7) gebildete U-förmige Ausnehmung (5), in welche der zu haltende Stab (4) einsetzbar ist, ein Verriegelungselement (15), mit welchem die U-förmige Ausnehmung (5) verschliessbar ist, eine Spannschraube (16) und ein Einsatzelement (9), das auf den Stab (4) aufsetzbar ist und diesen mindestens teilweise umschliesst, und der Stab (4) im eingespannten Zustand zwischen einer ersten Spannfläche (17) und einer zweiten Spannfläche (18) gehalten ist, und das Einsatzelement (9) mit Anschlagflächen (11) ausgestattet ist, durch welche beim Spannvorgang die durch die Spannschraube (16) auf das Einsatzelement (9) übertragene Spannbewegung gegen den Stab (4) hin begrenzt ist, wobei die erste Spannfläche (17) und/oder die zweite Spannfläche (18) mindestens teilweise mit Erhöhungen (27) und/oder mit Vertiefungen (28) ausgestattet sind und der in die Pedikelschraube (1) einsetzbare Stab (4) eine zu den Spannflächen (17, 18) mindestens teilweise komplementäre Oberfläche aufweist,
**dadurch gekennzeichnet, dass** die Breite der an den Spannflächen (17, 18) angebrachten Erhöhungen (27) in Stabrichtung gegen die Randbereiche hin abnehmend und/oder die Breite der an den Spannflächen (17, 18) angebrachten Vertiefungen (28) in Stabrichtung gegen die Randbereiche hin zunehmend sind.

2. Pedikelschraube mit einer Verschlusseinrichtung nach Anspruch 1, **dadurch gekennzeichnet, dass** das Einsatzelement (9) als Schiebeteil (10) ausgebildet ist, welches geführt zwischen die beiden Schenkel (6, 7) der U-förmigen Ausnehmung (5) einsetzbar ist, welche Schenkel (6, 7) mit jeweils einer innenseitig vorstehenden Schulter (13, 14) ausgestattet sind, auf welchen sich die Anschlagflächen (11, 12) des Schiebeteils (10) abstützen, und dass die erste Spannfläche (17) durch die gegen den Stab (4) gerichtete Fläche des Schiebeteils (10) und die zweite Spannfläche (18) durch den Grund der U-förmigen Ausnehmung (5) im Kopfteil (3) gebildet sind.

3. Pedikelschraube mit einer Verschlusseinrichtung nach Anspruch 1, **dadurch gekennzeichnet, dass** das Einsatzelement (9) aus einer im wesentlichen hohlzylinderförmigen Hülse (19) gebildet ist, in welche der Stab (4) einsetzbar ist, welche Hülse (19) aus einer ersten Halbschale (20) und einer zweiten Halbschale (21) zusammengesetzt ist und welche beiden Halbschalen (20, 21) über die Spannschraube (16) gegeneinander spannbar sind, wobei an der ersten Halbschale (20) die Anschlagflächen (11) angebracht sind, welche auf entsprechend an der zweiten Halbschale (21) angebrachte Stützflächen (13) abstützbar sind, wobei die Innenseite der ersten Halbschale (20) die erste Spannfläche (17) und die Innenseite der zweiten Halbschale (21) die zweite Spannfläche (18) bilden.

4. Pedikelschraube mit einer Verschlusseinrichtung nach Anspruch 3, **dadurch gekennzeichnet, dass** die erste Halbschale (20) und die zweite Halbschale (21) entlang der einen Trennlinie (22) gelenkig miteinander verbunden sind und dass die erste Halbschale (20) auf der aufschwenkbaren Seite mindestens eine Lasche (23) aufweist, welche in entsprechend an der aufschwenkbaren Seite der zweiten Halbschale (21) angebrachte Ausnehmungen (24) eingreift.

5. Pedikelschraube mit einer Verschlusseinrichtung nach einem der Ansprüche 1 bis 4, **dadurch gekennzeichnet, dass** die Erhöhungen (27) als Rippen und die Vertiefungen (28) als Rillen ausgebildet sind, welche Rippen und Rillen im Wesentlichen quer zur Stabrichtung verlaufen.

6. Pedikelschraube mit einer Verschlusseinrichtung nach einem der Ansprüche 1 bis 5, **dadurch gekennzeichnet, dass** der Stab (4) aus einem biokompatiblen Kunststoff auf Basis von Polyurethan und die Pedikelschraube (1) mit Verriegelungselement (15) und Einsatzelement (9) aus einer Titanlegierung bestehen.

7. Pedikelschraube mit einer Verschlusseinrichtung nach einem der Ansprüche 1 bis 6, **dadurch gekennzeichnet, dass** der in die Pedikelschraube (1) einzusetzende Stab (4) erwärmt wird.

## Claims

1. Pedicle screw with a closure device (8), in which a rod (4) made up of a flexible plastic is received and secured, for stabilization of a vertebral column, comprising a screw-in part (2), a head part (3) provided on the screw-in part (2), a U-shaped recess (5), formed by two arms (6, 7), disposed in the head part (3), into which recess the rod (4) to be held is insertable, a locking element (15) by means of which the U-shaped recess (5) is closable, a clamping screw (16) and an insert (9) which is placeable on the rod (4) and encloses the latter at least partially, and the rod (4) is held in clamped state between a first clamping surface (17) and a second clamping surface (18), and the insert (9) is provided with stop faces (11) by means of which the clamping action against the rod (4), transmitted to the insert (9) by the clamping screw (16) during the clamping step, is limited, the first clamping surface (17) and/or the second clamping surface (18) being provided at least partially with elevations (27) and/or with depressions (28), and the rod (4) inserted in the pedicle screw (1) having a surface at least partially complementary to the clamping surfaces (17, 18), **characterized in that** the width of the elevations (27) provided on the clamping surfaces (17, 18) decreases in rod direction toward the peripheral regions and/or the width of the depressions (28) provided on the clamping surfaces (17, 18) increases in rod direction toward the peripheral regions.

2. Pedicle screw with a closure device according to claim 1, **characterized in that** the insert (9) is designed as sliding part (10) which is insertable in a guided way between the two arms (6, 7) of the U-shaped recess (5), which arms (6, 7) are each provided with a shoulder (13, 14) protruding on the inside, on which shoulder the stop faces (11, 12) of the sliding part (10) support themselves, and **in that** the first clamping surface (17) is formed by the surface of the sliding part (10) directed toward the rod (4), and the second clamping surface (18) by the bottom of the U-shaped recess (5) in the head part (3).

3. Pedicle screw with a closure device according to claim 1, **characterized in that** the insert (9) is formed by a substantially hollow cylindrical sleeve (19) into which the rod (4) is insertable, which sleeve (19) is composed of a first half-shell (20) and a second half-shell (21) and which two half-shells (20, 21) are able to be clamped against each other via the clamping screw (16), the stop faces (11) being provided on the first half-shell (20) and being able to be supported on support surfaces (13) correspondingly provided on the second half-shell (21), the inner surface of the first half-shell (20) form ing the first clamping surface (17), and the inner surface of the second half-shell (21) the second clamping surface (18)

4. Pedicle screw with a closure device according to claim 3, **characterized in that** the first half-shell (20) and the second half-shell (21) are connected to each other in an articulated way along the one line of separation (22), and **in that** the first half-shell (20) has at least one tab (23) on the side able to be pivoted open, which tab engages in recesses (24) correspondingly provided on the side, able to be pivoted open, of the second half-shell (21).

5. Pedicle screw with a closure device according to one of the claims 1 to 4, **characterized in that** the elevations (27) are designed as ridges and the depressions (28) as grooves, which ridges and grooves run substantially transversely to the rod direction.

6. Pedicle screw with a closure device according to one of the claims 1 to 5, **characterized in that** the rod (4) consists of a polyurethane-based biocompatible plastic, and the pedicle screw (1) with locking element (15) and insert (9) of a titanium alloy.

7. Pedicle screw with a closure device according to one of the claims 1 to 6, **characterized in that** the rod (4) to be inserted in the pedicle screw (1) is heated.

## Revendications

1. Vis pédiculaire avec un dispositif de fermeture (8), dans laquelle une tige (4) en matériau plastique flexible est reçue et fixée, pour la stabilisation d'une colonne vertébrale, comprenant une partie de vissage (2), une partie de tête (3) prévue sur la partie de vissage (2), un évidement en forme de U (5), formé par deux bras (6, 7), agencé dans ladite partie de tête (3), évidement dans lequel ladite tige (4) devant être tenue est susceptible d'être insérée, un élément de verrouillage (15) par lequel ledit évidement en forme de U (5) est susceptible d'être fermé, une vis de serrage (16) et un insert (9) susceptible d'être placé sur ladite tige (4) et enserrant cette dernière au moins partiellement, et ladite tige (4) est tenue en état serré entre une première surface de serrage (17) et une seconde surface de serrage (18), et ledit insert (9) est muni de surfaces de butée (11) par lesquelles le mouvement de serrage contre ladite tige (4), transmis audit insert (9) par ladite vis de serrage (16) lors de l'étape de serrage, est limité, ladite première surface de serrage (17) et/ou ladite seconde surface de serrage (18) étant munies au moins partiellement de saillies (27) et/ou d'évidements (28), et ladite tige (4) insérée dans la vis pédiculaire (1) ayant une surface au moins partiellement complémentaire auxdites surfaces de serrages (17, 18), **caractérisée en ce que** la largeur desdites saillies (27) prévues sur lesdites surfaces de serrage (17, 18) diminue dans la direction de la tige vers les régions périphériques et/ou la largeur desdits évidements (28) prévus sur lesdites surfaces de serrage (17, 18) augmente dans la direction de la tige vers les régions périphériques.

2. Vis pédiculaire avec un dispositif de fermeture selon la revendication 1, **caractérisée en ce que** ledit insert (9) est façonné sous forme de pièce coulissante (10) susceptible d'être insérée de manière guidée entre les deux bras (6, 7) dudit évidement en forme de U (5), bras (6, 7) dont chacun est muni d'un épaulement (13, 14) faisant saillie du côté intérieur, épaulement sur lequel les surfaces de butée (11, 12) de ladite pièce coulissante (10) sont supportées, et **en ce que** ladite première surface de serrage (17) est formée par la surface de ladite pièce coulissante (10) dirigée vers ladite tige (4), et ladite seconde surface de serrage (18) est formée par le fond dudit évidement en forme de U (5) dans ladite partie de tête (3).

3. Vis pédiculaire avec un dispositif de fermeture selon la revendication 1, **caractérisée en ce que** ledit insert (9) est formé par une douille sensiblement creuse et cylindrique (19) dans laquelle ladite tige (4) est susceptible d'être insérée, ladite douille (19) étant composée d'une première demi-douille (20) et d'une seconde demi-douille (21), lesdites deux demi-douilles (20, 21) étant susceptibles d'être serrées l'une contre l'autre moyennant ladite vis de serrage (16), ladite première demi-douille (20) étant munie desdites surfaces de butée (11) susceptibles d'être supportées sur des surfaces de support (13) prévues de manière correspondante sur ladite seconde demi-douille (21), la surface interne de ladite première demi-douille (20) formant ladite première surface de serrage (17), et la surface interne de ladite seconde demi-douille (21) formant ladite seconde surface de serrage (18).

4. Vis pédiculaire avec un dispositif de fermeture selon la revendication 3, **caractérisée en ce que** ladite première demi-douille (20) et ladite seconde demi-douille (21) sont raccordées l'une à l'autre de manière articulée le long de la ligne de séparation (22), et **en ce que** ladite première demi-douille (20) a au moins une patte (23) du côté susceptible d'être ouvert en pivotant, ladite patte venant en prise avec des évidements (24) prévus de manière correspondante du côté, susceptible d'être ouvert en pivotant, de ladite seconde demi-douille (21).

5. Vis pédiculaire avec un dispositif de fermeture selon l'une des revendications 1 à 4, **caractérisée en ce que** lesdites saillies (27) sont façonnées sous forme de nervures et lesdits évidements (28) sous forme de rainures, lesdites nervures et rainures s'étendant sensiblement transversalement à la direction de la tige.

6. Vis pédiculaire avec un dispositif de fermeture selon l'une des revendications 1 à 5, **caractérisée en ce que** ladite tige (4) consiste en matériau plastique biocompatible à base de polyuréthane, et la vis pédiculaire (1) avec ledit élément de verrouillage (15) et ledit insert (9) en alliage de titane.

7. Vis pédiculaire avec un dispositif de fermeture selon l'une des revendications 1 à 6, **caractérisée en ce que** ladite tige (4) devant être insérée dans la vis pédiculaire (1) est chauffée.
